# EUROPEAN PATENT APPLICATION

(11) **EP 4 706 392 A1**
(43) Date of publication of application: **11.03.2026**
(21) Application number: 24888831.5
(22) Date of filing: 08.11.2024
(51) Int. Cl.: A01N 59/00, A01N 25/02, A01P 7/02, A01P 7/04

(54) **OZONE-CONTAINING COMPOSITION AND METHOD FOR PRODUCING OZONE-CONTAINING COMPOSITION**

(30) Priority: 10.11.2023 JP 2023192158
(71) Applicant: E-Tech Co., Ltd., Kobe city, Hyogo 654-0151 (JP)
(72) Inventor: YOSHIDA, Eiichi, Kobe city, Hyogo 654-0151 (JP)
(74) Representative: Valet Patent Services Limited
(86) International application number: PCT/JP2024/039881
(87) International publication number: WO 2025/100540

(57) **Abstract**

An ozone-containing composition comprises a volatile oily solvent, a volatile aqueous solvent, and ozone. The volatile oily solvent is present in a concentration ranging from 1 wt% to 99 wt% based on the total composition. The volatile aqueous solvent is present in a concentration ranging from 1 wt% to 99 wt% based on the total composition. The volatile oily solvent is a volatile liquid paraffin having a boiling point of 170 degrees and being lipophilic, a volatile silicone oil having a boiling point ranging from 170 degrees to 200 degrees and being lipophilic, or a mixture of the volatile liquid paraffin and the volatile silicone oil. The volatile liquid paraffin is one or more volatile liquid paraffins selected from the group consisting of light isoparaffin, light liquid isoparaffin, and hydrogenated polyisobutene. The volatile silicone oil is a volatile silicone oil containing cyclic dimethyl silicone oil. The volatile aqueous solvent is a volatile alcohol having a boiling point of 150 degrees or less and being hydrophilic and lipophilic, a volatile ketone having a boiling point of 150 degrees or less and being hydrophilic and lipophilic, or a mixture of the volatile alcohol and the volatile ketone. The volatile alcohol comprises ethanol, isopropyl alcohol, or 1-pentanol. The volatile ketone comprises methyl ethyl ketone, or 2-hexanone. The ozone-containing composition is for use in killing insects, repelling pests, or decontaminating and decomposing anticancer drugs.

## Description

### [TECHNICAL FIELD]

The present invention relates to an ozone-containing composition and a method for producing the ozone-containing composition.

### [BACKGROUND ART]

Ozone (ozone gas) has been known to have a very strong bactericidal power, and the power is due to a destruction of bacterial cell membranes or nucleic acids. Therefore, ozone is capable of inactivating (killing) all bacteria, including highly durable spore-forming bacteria compared with general disinfectants and the ozone has a characteristic of not generating resistant bacteria.

The present inventor has independently developed disinfectants and germicides containing ozone by utilizing such characteristic of ozone, filed patent applications, and obtained rights. For example, Japanese Patent Laid-Open Publication No. 2009-525974 (Patent Document 1) discloses a disinfectant containing ozone, which is a disinfectant that is given bactericidal power by mixing ozone into liquid paraffin or Vaseline while stirring the mixture to make the ozone contained therein. In addition, Japanese Unexamined Patent Publication No. 2013-234132 (Patent Document 2) discloses an ozone-containing silicone oil that is given bactericidal power by blowing ozone into silicone oil as bubbles while stirring the silicone oil, thereby making the silicone oil contain ozone as fine bubbles. Furthermore, Japanese Unexamined Patent Publication No.2015-071586 (Patent Document 3) discloses a volatile disinfectant which is obtained by stirring a volatile solvent containing volatile liquid paraffin, volatile silicone oil, or a mixture thereof as a base while blowing ozone gas into the volatile solvent, thereby making the volatile solvent to contain ozone gas, and when applied to a specified object, the volatile disinfectant inactivates pathogens on the object and then volatilizes from the object.

On the other hand, in addition to the use of disinfection and bactericide, the above-mentioned characteristics of ozone are being used to develop pest control and insecticide, and to decontaminate and decompose anticancer drugs used in medical settings. For example, Japanese Unexamined Patent Publication No.Hei-05-170610 (Patent Document 4) discloses a method for exterminating pests by exposure to ozone. It is said that household pests and pests called nuisance pests, such as cockroaches, mites, termites, fleas, lice, flies, and mosquitoes, can be exterminated by exposure to ozone at a concentration of 0.2 ppm or more, or to the ozone and a trace amount of nitrogen oxides. It is also said that pest extermination can be performed efficiently by a pest extermination device equipped with an ozone generating means and an ozone decomposition means.

Japanese Unexamined Patent Publication No.2019-208864 (Patent Document 5) discloses a sterilization or pest control system for sterilizing or pest control fruits and vegetables by exposing the fruits and vegetables to a sterilizing or pest control gas having a sterilizing or pest control effect in a gas-filled space. This system includes a memory unit, a selection designation receiving unit, a determination processing unit, and a control processing unit. The memory unit stores a combination of the gas concentration of the sterilizing or pest control gas and the exposure time of the fruits and vegetables to the sterilizing or pest control gas as a sterilization or pest control condition for each type of fruits and vegetables. The selection designation receiving unit receives the selection of the type of fruits and vegetables and the designation of the exposure time or gas concentration. The determination processing unit reads out the sterilization or pest control condition corresponding to the type of fruits and vegetables selected by the selection designation receiving unit from the memory unit, and determines the gas concentration corresponding to the exposure time designated by the selection designation receiving unit, or the exposure time corresponding to the gas concentration designated by the selection designation receiving unit. The control processing unit generates a sterilizing or pest control gas and maintains the gas concentration in the gas-filled space determined by the determination processing unit, or controls the exposure of the fruits or vegetables to the sterilizing or pest control gas for the exposure time determined by the determination processing unit. The sterilizing or pest control gas is ozone gas. In this way, it is possible to obtain a sufficient sterilizing or pest control effect at a gas concentration or exposure time that does not cause damage to the fruits or vegetables by using a sterilizing or pest control gas that has a sterilizing or pest control effect on the fruits or vegetables.

Republication of Japanese Patent Application No. 2019-073996 (Patent Document 6) discloses a harmful substance treatment method in which ozone gas is applied to decompose or sterilize harmful substances in an environment humidified with an aqueous surfactant solution. The harmful substances are anticancer drugs, and examples of the surfactants used include nonionic surfactants, anionic surfactants, cationic surfactants, and amphoteric surfactants. In this way, it is possible to decompose harmful substances that are not easily decomposed by ozone gas alone, or of promoting the detoxification of harmful microorganisms.

Republication of Japanese Patent Application No. 2014-208428 (Patent Document 7) discloses a method for decomposing an anticancer drug by using air containing ozone and humidified by a humidifying means to decompose the anticancer drug. In this way, it is possible to protect medical staff from anticancer drugs that are scattered outside (safety cabinets, dispensing rooms, or the like) during dispensing, or the like.

### [CITATION LIST]

### [PATENT LITERATURE]

[PTL1] Japanese Patent Laid-Open Publication No. 2009-525974
[PTL2] Japanese Unexamined Patent Publication No. 2013-234132
[PTL3] Japanese Unexamined Patent Publication No.2015-071586
[PTL4] Japanese Unexamined Patent Publication No.Hei-05-170610
[PTL5] Japanese Unexamined Patent Publication No.2019-208864
[PTL6] Republication of Japanese Patent Application No. 2019-073996
[PTL7] Republication of Japanese Patent Application No. 2014-208428

### [SUMMARY OF INVENTION]

### [TECHNICAL PROBLEM]

By the way, gas, liquid, and solid chemicals are used to kill and exterminate pests, but these chemicals usually have a problem that they have a bad effect on the human body. Also, chemicals usually have a residual effect, so there is a problem that the chemicals remaining after application have a bad effect on the external environment. Furthermore, even if ozone is sprayed on pests, there are cases where the pests do not die, and there is a problem that ozone alone is not useful for killing and exterminating pests.

The same is true for the decontamination and decomposition of anticancer drugs handled in medical settings. Although anticancer drugs have a pharmacological effect on patients when administered appropriately, they may cause health problems for medical professionals other than patients, such as doctors, nurses, and pharmacists. For example, medical professionals may develop health problems due to inhalation exposure to vaporized anticancer drugs or percutaneous exposure due to contact with leaked anticancer drugs, causing acute symptoms such as gastrointestinal symptoms and circulatory symptoms, or leading to the onset of cancer due to the chronic toxicity (carcinogenicity, or the like) of anticancer drugs. Therefore, in actual situations, medical professionals carefully prepare, administer, and dispose of anticancer drugs according to prescribed procedures, but there is a problem that these measures are not sufficient. In addition, there is a problem that the processing agents used for decontamination and decomposition of general anticancer drugs remain on the anticancer drugs after application or contaminate an object of application. Here, the technology described in Patent Documents 1-7 cannot solve the above-mentioned problems.

Therefore, the present invention has been made to solve the above problems, and aims to provide an ozone-containing composition and a method for producing an ozone-containing composition that can obtain an insecticidal effect, an insect pest repellent effect, and an effect of decontaminating and decomposing an anticancer drug, can be safe for the human body, and can be easily post-treated after application.

### [SOLUTION TO PROBLEM]

An ozone-containing composition in the present invention comprises a volatile oily solvent, a volatile aqueous solvent, and ozone. The volatile oily solvent is present in a concentration ranging from 1 wt% to 99 wt% based on the total composition. The volatile aqueous solvent is present in a concentration ranging from 1 wt% to 99 wt% based on the total composition. The volatile oily solvent is a volatile liquid paraffin having a boiling point of 170 degrees and being lipophilic, a volatile silicone oil having a boiling point ranging from 170 degrees to 200 degrees and being lipophilic, or a mixture of the volatile liquid paraffin and the volatile silicone oil. The volatile liquid paraffin is one or more volatile liquid paraffins selected from the group consisting of light isoparaffin, light liquid isoparaffin, and hydrogenated polyisobutene. The volatile silicone oil is a volatile silicone oil containing cyclic dimethyl silicone oil. The volatile aqueous solvent is a volatile alcohol having a boiling point of 150 degrees or less and being hydrophilic and lipophilic, a volatile ketone having a boiling point of 150 degrees or less and being hydrophilic and lipophilic, or a mixture of the volatile alcohol and the volatile ketone. The volatile alcohol comprises ethanol, isopropyl alcohol, or 1-pentanol. The volatile ketone comprises methyl ethyl ketone, or 2-hexanone. The ozone-containing composition in the present invention is for use in killing insects, repelling pests, or decontaminating and decomposing anticancer drugs.

The method for producing the ozone-containing composition in the present invention comprises a mixing step and a stirring step. The mixing step involves introducing a volatile oily solvent, a volatile aqueous solvent, and ozone into a predetermined mixing tank as a mixture of the ozone-containing composition and mixing them. The stirring step involves stirring the mixture in the mixing tank using a predetermined stirrer.

### [ADVANTAGEOUS EFFECTS OF THE INVENTION]

According to the present invention, it is possible to obtain an insecticidal effect, an insect pest repellent effect, and an effect of decontaminating and decomposing an anticancer drug, to be safe for the human body, and to be easily post-treated after application.

### [BRIEF DESCRIPTION OF THE DRAWINGS]

[FIG. 1]

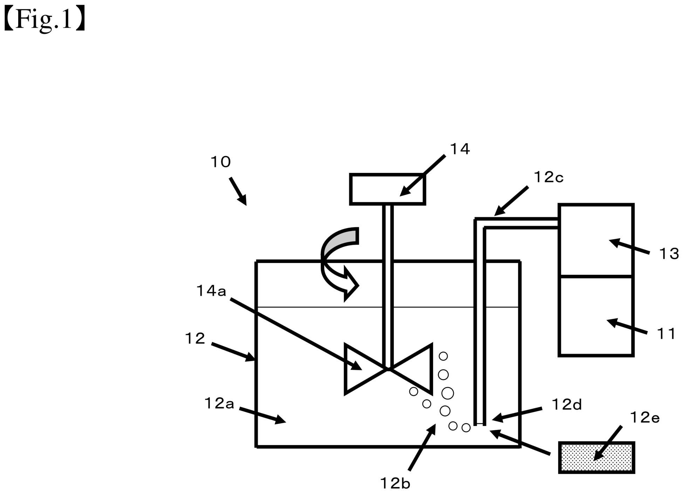

   FIG.1 is a conceptual diagram illustrating a method for producing an ozone-containing composition according to the present invention.
[FIG. 2]

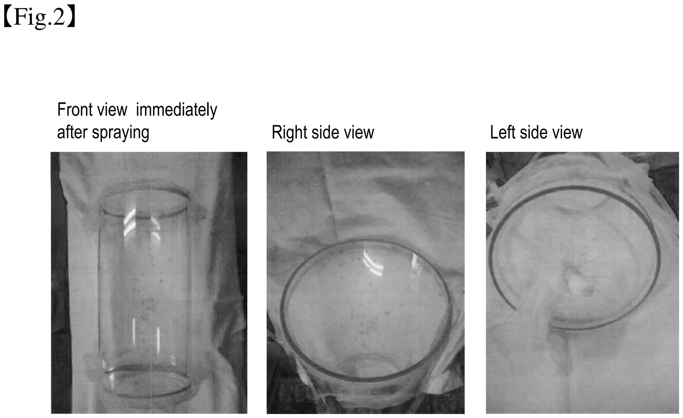

   FIG.2 is a front photograph, a right side photograph and a left side photograph, respectively, taken immediately after spraying about an example of the results of the mosquito insecticidal test in Example 1.
[FIG. 3]

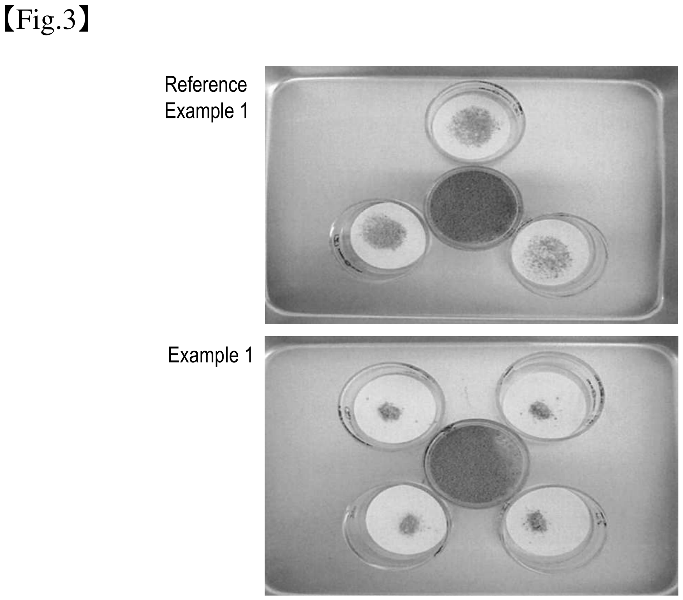

   FIG.3 is a photograph showing an example of the results of the anti-mite test in Reference Example 1 and Example 1.
[FIG. 4]

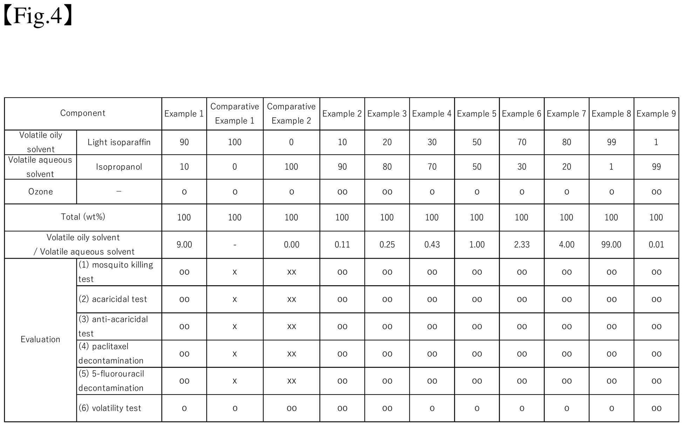

   FIG.4 is a table showing the components and evaluation results of the ozone-containing compositions of Examples 1 to 9 and Comparative Examples 1 and 2.
[FIG. 5]

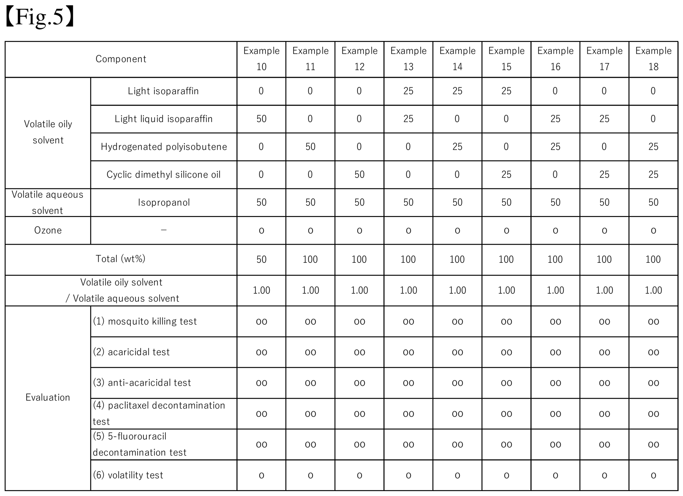

   FIG. 5 is a table showing the components and evaluation results of the ozone -containing compositions in Examples 10 to 18.
[FIG. 6]

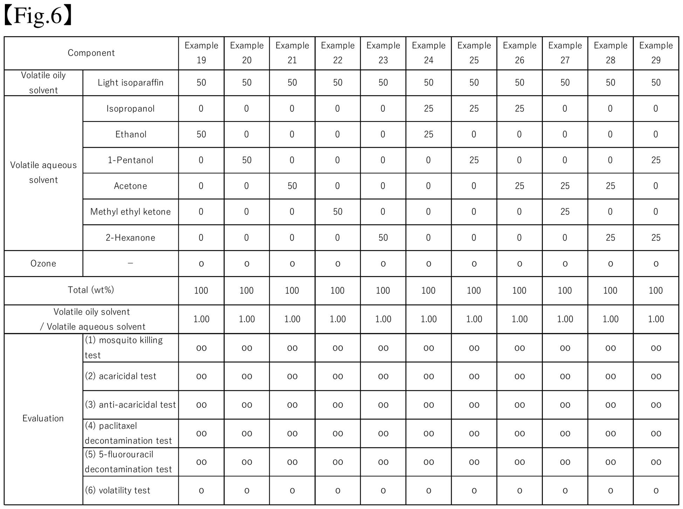

   FIG. 6 is a table showing the components and evaluation results of the ozone-containing compositions in Examples 19 to 29.
[FIG. 7]

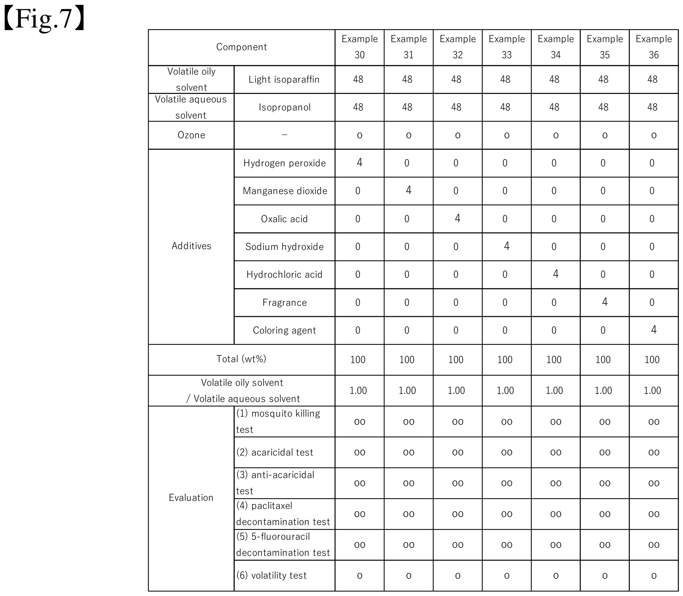

   FIG. 7 is a table showing the components and evaluation results of the ozone-containing compositions in Examples 30 to 36.
[FIG. 8]

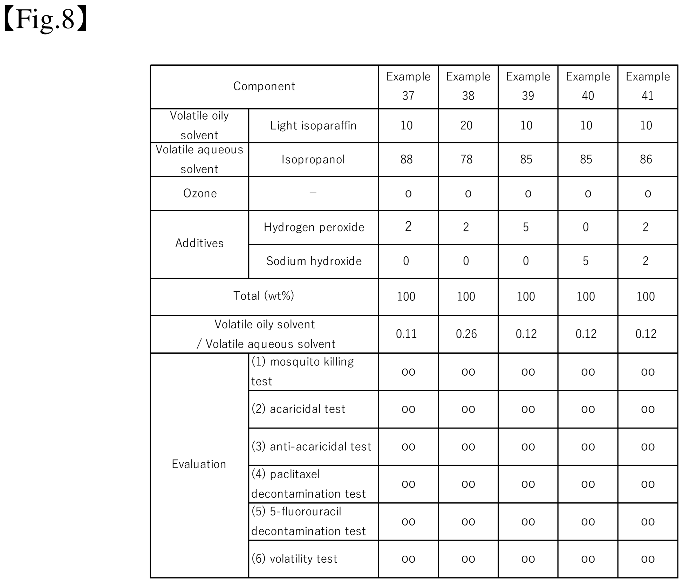

   FIG. 8 is a table showing the components and evaluation results of the ozone-containing compositions in Examples 37 to 41.
[FIG. 9]

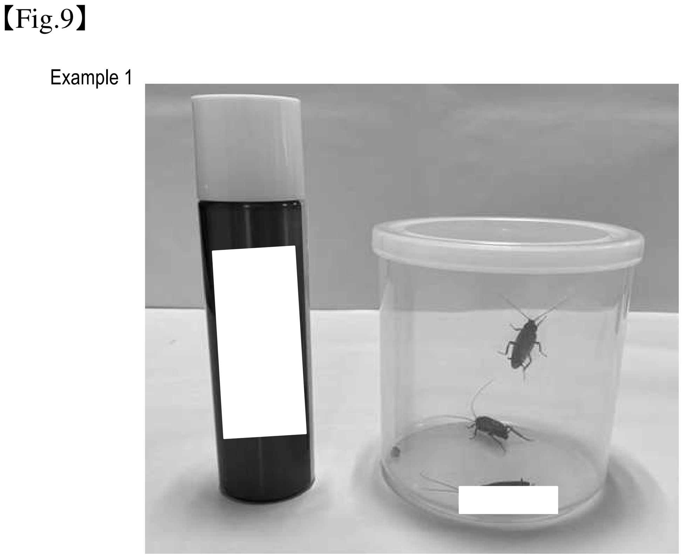

   FIG.9 is a photograph showing an example of a cockroach insecticidal test in Example 1.

### [DESCRIPTION OF EMBODIMENTS]

The preferred embodiments of the present invention will be explained below according to the attached drawings; thereby the present invention will be clearly understood. The embodiments below are examples materializing the present invention, and do not limit the technical scope of the present invention.

The present inventor has been actively studying disinfectants and germicides containing ozone for many years. Such disinfectants and germicides basically dissolve ozone in an oily solvent, retain the ozone in the oily solvent, and bring the oily solvent into contact with bacteria, whereby the ozone in the oily solvent comes into contact with the bacteria and inactivates the bacteria.

Here, the inventor tried to apply such disinfectants and germicides for killing and repelling pests, but it has been found that pests are surprisingly highly hydrophilic, and even if an oily solvent in which ozone is dissolved is applied to pests, it is difficult to obtain the desired killing and repelling effects. Here, insects such as pests do not have lungs, and breathe by directly exchanging oxygen and carbon dioxide through a trachea, but even if ozone is sprayed on pests, the ozone does not come into contact with the trachea of the pests appropriately, so it does not hinder the respiration of the pests, and it has been found that it is difficult to kill or repel pests with ozone alone.

The present inventors also attempted to decontaminate and decompose anticancer drugs, and found that, as described above, anticancer drugs also have hydrophilic properties, and therefore, even if an oily solvent in which ozone is dissolved is applied to the anticancer drug, it is difficult to obtain a sufficient effect of decontaminating and decomposing the anticancer drug.

Therefore, the inventor added a certain amount of an aqueous solvent that can be mixed with the oily solvent, and surprisingly discovered that the ozone in the oily solvent contacted the trachea of pests through the aqueous solvent, resulting in an insecticidal effect and an insect repellent effect. The same was true for decontamination and decomposition of anticancer drugs, and the inventor discovered that the ozone in the oily solvent contacted the anticancer drug through the aqueous solvent, resulting in decontamination and decomposition of the anticancer drug. As a result, the inventor completed the present invention based on Examples described below.

Namely, the ozone-containing composition according to the present invention comprises a volatile oily solvent, a volatile aqueous solvent, and ozone. The volatile oily solvent is present in a concentration ranging from 1 wt% to 99 w % based on the total composition. The volatile aqueous solvent is present in a concentration ranging from 1 wt% to 99 wt% based on the total composition. The volatile oily solvent is a volatile liquid paraffin having a boiling point of 170 degrees and being lipophilic, a volatile silicone oil having a boiling point ranging from 170 degrees to 200 degrees and being lipophilic, or a mixture of the volatile liquid paraffin and the volatile silicone oil. The volatile liquid paraffin is one or more volatile liquid paraffins selected from the group consisting of light isoparaffin, light liquid isoparaffin, and hydrogenated polyisobutene. The volatile silicone oil is a volatile silicone oil containing cyclic dimethyl silicone oil. The volatile aqueous solvent is a volatile alcohol having a boiling point of 150 degrees or less and being hydrophilic and lipophilic, a volatile ketone having a boiling point of 150 degrees or less and being hydrophilic and lipophilic, or a mixture of the volatile alcohol and the volatile ketone. The volatile alcohol comprises ethanol, isopropyl alcohol, or 1-pentanol. The volatile ketone comprises methyl ethyl ketone, or 2-hexanone. The ozone-containing composition in the present invention is for use in killing insects, repelling pests, or decontaminating and decomposing anticancer drugs.

Here, volatile liquid paraffin and volatile silicone oil are lipophilic and stable against ozone (do not oxidize). In addition, the boiling point of volatile liquid paraffin is 170 degrees, and the boiling point of volatile silicone oil is in the range of 170 degrees to 200 degrees, so both volatile liquid paraffin and volatile silicone oil evaporate and dry when exposed to the atmosphere for a while. The SP value {(cal/cm3)1/2} of volatile liquid paraffin is within the range of 6.5 to 7.5, and the SP value {(cal/cm3)1/2} of volatile silicone oil is within the range of 6.5 to 9.5, and the SP value of volatile liquid paraffin and the SP value of volatile silicone oil overlap in part, so that volatile liquid paraffin is mixed uniformly with volatile silicone oil without reacting with it. Here, the SP value (cal/cm3)1/2 is defined as (evaporation energy/molar volume)1/2, which is a parameter reflecting the cohesive force of a substance, and means that solvents with close or overlapping SP values have good compatibility. The above SP value is an estimated value by the Fedors method. In the present invention, ozone is dissolved in such a volatile oily solvent.

On the other hand, the volatile alcohol and volatile ketone of the volatile aqueous solvent are hydrophilic and lipophilic. Here, the volatile alcohol means an alcohol with a boiling point of 150 degrees or less, and the volatile ketone means a ketone with a boiling point of 150 degrees or less. The SP value {(cal/cm3)1/2} of the volatile alcohol is in the range of 10.0 to 13.8, and the SP value {(cal/cm3)1/2} of the volatile ketone is in the range of 8.0 to 10.0. That is, the SP value of the volatile alcohol and the SP value of the volatile ketone overlap partially, and also overlap partially with the SP value of the volatile liquid paraffin and the SP value of the volatile silicone oil. The volatile alcohol is mixed uniformly without reacting with the volatile ketone, and the volatile alcohol and the volatile ketone are mixed uniformly without reacting with the volatile liquid paraffin and the volatile silicone oil. Furthermore, the volatile alcohol and the volatile ketone have a lower reactivity with ozone than carboxylic acids and fatty acids. Therefore, in the present invention, by mixing a volatile aqueous solvent with a volatile oily solvent and ozone, the hydrophilic properties are imparted to the whole, which increases the contact with the trachea of pests and the anticancer drug, and it is possible to effectively contact the ozone with the anticancer drug and pests, and it is possible to obtain an insecticidal effect, a pest repellent effect, and an anticancer drug decontamination and decomposition effect. In addition, since the boiling point of the volatile aqueous solvent is lower than that of the volatile oily solvent, the volatile aqueous solvent volatilizes first, and then the volatile oily solvent containing ozone volatilizes, and it is possible to appropriately contact the ozone with the trachea of pests and the anticancer drug.

Here, the volatile liquid paraffin and volatile silicone oil of the volatile oily solvent are used in cosmetics, food additives, or the like, and are harmless to the human body even if the user inhales them. In addition, ozone is approved as a food additive by the U.S. Food and Drug Administration (FDA), and when it is at a low concentration, it is harmless to the human body even if the user inhales it. Furthermore, since the volatile alcohols and volatile ketones in the volatile aqueous solvents are unlikely to affect the human body, it is possible to ensure safety overall. Therefore, according to the present invention, it is possible to achieve insecticidal effects, pest repellent effects, and effects such as decontamination and decomposition of anticancer drugs without harming the health of the users handling it.

Here, the volatile oily solvent does not react with ozone, and the volatile aqueous solvent hardly reacts with ozone when mixed with the volatile oily solvent. Therefore, for example, it is possible to store the present ozone-containing composition for a long period of time by sealing it.

In this way, the ozone-containing composition according to the present invention can suitably obtain insecticidal effects, pest repellent effects, effects of decontamination and decomposition of anticancer drugs, or the like, in the fields of hygiene, medicine, agriculture, dairy farming, or the like. In addition, it is possible to apply the composition to other uses and fields that require decomposition of hydrophilic objects by ozone.

Here, the type of ozone is not particularly limited, and examples thereof include a mixed gas of ozone and oxygen, a single gas of ozone only, or the like. The mixture ratio of ozone and oxygen in the mixed gas is, for example, in the range of 50 wt% to 2 wt% for ozone and in the range of 50 wt% to 98 wt% for oxygen.

In addition, the concentration of ozone in the present ozone-containing composition is not particularly limited, for example, from the viewpoints of the reliability of the insecticidal effect, pest repellent effect, decontamination/decomposition effect of anticancer drugs, and safety to the human body, the concentration is preferably in the range of 0.1 ppm to 5000.0 ppm, more preferably in the range of 0.1 ppm to 2500.0 ppm, and even more preferably in the range of 0.1 ppm to 100.0 ppm. This makes it possible to appropriately adjust the concentration of ozone in the atmosphere that evaporates together with the volatile solvent, thereby ensuring safety to the human body. The ozone concentration in the gas and the ozone concentration in the composition (solvent) of the present invention are completely different.

In addition, the type of volatile liquid paraffin of the volatile oily solvent is not particularly limited, and examples thereof include light isoparaffins such as isododecane and isohexadecane, light liquid isoparaffins (a mixture of hydrocarbons having side chains obtained by copolymerizing isobutene and n-butene and then hydrogenating them), and hydrogenated polyisobutene (a branched aliphatic hydrogen oxide, a synthetic liquid oil mainly composed of isoparaffin). In addition, the type of volatile silicone oil of the volatile oily solvent is not particularly limited, and examples thereof include cyclic dimethyl silicone oil. In addition, the composition of the volatile liquid paraffin is not particularly limited, and it may be one type of volatile liquid paraffin or a mixture of two or more types of volatile liquid paraffins. In addition, the composition of the volatile silicone oil is not particularly limited, and it may be one type of volatile silicone oil or a mixture of two or more types of volatile silicone oils. The volatile liquid paraffin and volatile silicone oil described in this specification include both of these meanings.

The type of volatile alcohol of the volatile aqueous solvent is not particularly limited, and includes alcohols having a boiling point of 150 degrees or less. For example, the volatile alcohols include methanol (boiling point of about 64 degrees), ethanol (boiling point of about 78 degrees), isopropyl alcohol (boiling point of about 80 degrees), tert-butyl alcohol (boiling point of about 82 degrees), allyl alcohol (boiling point of about 97 degrees), 1-propanol (boiling point of about 97 degrees), 1-propyl alcohol (boiling point of about 97 degrees), 2-butanol (boiling point of about 100 degrees), 2-methyl-2-butanol (boiling point of about 102 degrees), 3-pentanol (boiling point of about 116 degrees), 1-butanol (boiling point of about 118 degrees), 2-methoxyethanol (boiling point of about 124 degrees), 2-chloroethanol (boiling point of about 128 degrees), 3-methyl-1-butanol (boiling point of about 131 degrees), isoamyl alcohol (boiling point of about 131 degrees), isopentyl alcohol (boiling point of about 131 degrees), propylene glycol monoethyl ether (boiling point of about 133 degrees), 2-ethoxyethanol (boiling point of about 135 degrees), 1-pentanol (boiling point of about 138 degrees), and propylene glycol monopropyl ether (boiling point of about 150 degrees).

Furthermore, the type of volatile ketone of the volatile aqueous solvent is not particularly limited, and includes ketones having a boiling point of 150 degrees or less. For example, the volatile ketones include acetone (2-propanone) (boiling point of about 56 degrees), methyl ethyl ketone (2-butanone) (boiling point of about 80 degrees), methyl vinyl ketone (3-buten-2-one) (boiling point of about 81 degrees), methyl isopropyl ketone (3-methyl-2-butanone) (boiling point of about 93 degrees), diethyl ketone (3-pentanone) (boiling point of about 101 degrees), methyl-n-propyl ketone (2-pentanone) (boiling point of about 102 degrees), methyl-n-butyl ketone (2-hexanone) (boiling point of about 128 degrees), acetylacetone (boiling point of about 141 degrees), di-n-propyl ketone (boiling point of about 144 degrees), and ethyl-n-butyl ketone (boiling point of about 148 degrees).

The concentration of the volatile oily solvent is not particularly limited as long as it is within the range of 1 wt% to 99 wt% of the total composition, but the volatile oily solvent is more preferably present in a concentration ranging from 10 wt% to 90 wt% based on the total composition, and even more preferably present in a concentration ranging from 10 wt% to 70 wt% based on the total composition. It is possible to increase the amount of ozone dissolved in the volatile oily solvent and thereby obtain the effect of ozone.

The concentration of the volatile aqueous solvent is not particularly limited as long as it is within the range of 1 wt% to 99 wt% based on the total composition, but the volatile aqueous solvent is more preferably present in a concentration ranging from 10 wt% to 90 wt% based on the total composition, and even more preferably present in a concentration ranging from 30 wt% to 90 wt% based on the total composition. It is possible to promote the volatility of the volatile aqueous solvent and to obtain the ozone-containing composition with excellent volatility.

] In addition, the mixing weight ratio (-) of the volatile oily solvent to the volatile aqueous solvent is not particularly limited. Here, when the mixing weight ratio (volatile oily solvent/volatile aqueous solvent) of the volatile oily solvent to the volatile aqueous solvent is close to 99.00, the amount of volatile oily solvent is large relative to the volatile aqueous solvent, so that the amount of solvent that dissolves ozone is large, resulting in the ozone-containing composition with high oxidizing power by ozone. On the other hand, when the mixing weight ratio of the volatile oily solvent to the volatile aqueous solvent is close to 0.01, the amount of volatile oily solvent is small relative to the volatile aqueous solvent, so that the volatile aqueous solvent promotes volatility, resulting in the ozone-containing composition with excellent volatility. When an insecticidal effect, a pest repellent effect, or an effect such as decontamination/decomposition of an anticancer drug is expected, the mixing weight ratio (-) of the volatile oily solvent to the volatile aqueous solvent is preferably, for example, within the range of 0.01 to 99.00, more preferably within the range of 0.4 to 15.0, and even more preferably within the range of 0.4 to 10.0. It is possible to increase the amount of ozone dissolved in volatile oily solvents and obtain the effect of ozone.

The ozone-containing composition of the present invention may further comprise additives, within a range that does not hinder the advantageous effects of the present invention, in addition to the above-mentioned volatile oily solvent, volatile aqueous solvent, and ozone. For Example, the additives include oxidizing agents, reducing agents, alkaline agents, acidic agents, fragrances, solvents, dyes, stabilizers, pH adjusters, ultraviolet light inhibitors, and antioxidants.

Here, the type of additive is not particularly limited, and examples thereof include oxidizing agents such as hydrogen peroxide, manganese dioxide, and nitric acid, reducing agents such as iron (II) ions, lithium aluminum hydride, sulfite, oxalic acid, and formic acid, alkaline agents such as sodium hydroxide, acidic agents such as hydrochloric acid, non-volatile liquid paraffin, non-volatile silicone oil, cyclomethyl hydrogen silicone oil, hydrocarbons, isoparaffins, mineral spirits, acyclic hydrocarbons, cyclic hydrocarbons, halogenated hydrocarbons, ethers, esters, and carboxylic acids. For Example, the acyclic hydrocarbons include pentane, hexane, heptane, octane, nonane, decane, undecane, and dodecane.The cyclic hydrocarbons include cyclopentane, cyclohexane, cycloheptane, and cyclooctane. The halogenated hydrocarbons include methylene chloride, ethyl chloride, chloroform, carbon tetrachloride, and trichloroethylene. The ethers include dimethyl ether, diethyl ether, butyl ether, and tetrahydrofuran (THF). The ester include methyl acetate, ethyl acetate, n-butyl acetate, γ-butyrolactone, or the like. The carboxylic acid include acetic acid, or the like. Also, examples of the solvent include solvents containing a double bond such as isophorone and oleic acid.

Here, from the viewpoint of increasing the oxidizing power of ozone, examples of the additive include alkaline agents such as hydrogen peroxide and sodium hydroxide. The concentration of the additive is set within a range that does not impair the effects of the present invention, and is preferably within a range of 0.1 wt% to 10.0 wt% based on the total composition, more preferably within a range of 1.0 wt% to 5.0 wt% based on the total composition, and even more preferably within a range of 1.0 wt% to 3.0 wt% based on the total composition. The additive is particularly preferably hydrogen peroxide alone, but may be an alkaline agent alone, or may be a mixture of hydrogen peroxide and an alkaline agent. The presence of additives containing hydrogen peroxide is expected to strengthen and sustain the oxidizing power of ozone.

In addition, the mode of use of the ozone-containing composition according to the present invention is not particularly limited. Here, since both the volatile oily solvent and the volatile aqueous solvent have high fluidity, the ozone-containing composition may be directly applied to the object, sprayed, or immersed. The ozone-containing composition may be impregnated into a cloth such as gauze and then attached to the object. Furthermore, the ozone-containing composition may be filled into an aerosol container (spray container) together with a high-pressure gas (compressed gas, liquefied gas) and used as an aerosol product. The ozone-containing composition may also be diluted and used as a dilution.

Furthermore, the type of pest to which the ozone-containing composition according to the present invention is applied is not particularly limited, and it is applicable to general pests. The pest may be such as mosquitoes, mites, fleas, lice, flies, bees, horseflies, cockroaches, ants, spiders, stink bugs, or the like.

The type of anticancer drug to which the ozone-containing composition according to the present invention is applied is not particularly limited, and it is applicable to general anticancer drugs. Examples of anticancer drugs include alkylating agents, metabolic antagonists, platinum compounds, topoisomerase inhibitors, microtubule inhibitors, and antibiotics. Examples of alkylating agents include nitrogen mustards such as cyclophosphamide, ifosfamide, busulfan, and melphalan, and nitrosoureas such as nimustine, ranimustine, carmustine, and streptozocin. Examples of metabolic antagonists include pyrimidine antagonists such as 5-fluorouracil, gemcitabine, cytarabine, and tegafur, purine antagonists such as 6-mercaptopurine, fludarabine, nelarabine, and pentostatin, and folate antagonists such as methotrexate and pemtrix sodium. Examples of platinum compounds include cisplatin, carboplatin, miriplatin, oxaliplatin, and nedaplatin. Examples of topoisomerase inhibitors include etoposide, irinotecan, nogitecan, and sobuzoxane. Examples of microtubule inhibitors include taxanes such as paclitaxel and docetaxel, vin alkaloids such as vincristine, vinblastine, and vindesi, and eribulins such as eribulin mesylate. Examples of antibiotics include anthracyclines such as doxorubicin, mitomycin, and daunomycin, actinomycin D, and bleomycin.

Next, the producing apparatus and producing method of the ozone-containing composition according to the present invention will be described. As shown in FIG. 1, the producing apparatus 10 of the ozone-containing composition according to the present invention includes a generation unit 11, a mixing tank 12, a supply unit 13, and an agitator 14. The generation unit 11 generates ozone. The mixing tank 12 contains both volatile solvents 12a, which are a volatile oily solvent and a volatile aqueous solvent. The supply unit 13 blows ozone 12b into the volatile solvent 12a in the mixing tank 12. The agitator 14 stirs the volatile solvent 12a into which the ozone 12b is being blown, thereby causing the volatile solvent 12a to contain the ozone 12b.

Here, for example, the method by which the generator unit 11 generates the ozone 12b may be, a silent discharge method, an electrolysis method, a photoreaction method, a highfrequency electrolysis method, a solid polymer electrolysis method, or the like. Also, an ozone cylinder containing the ozone 12b may be used as the generator unit 11 as it is.

The method of blowing in ozone 12b by the supply unit 13 can be, for example, as shown in FIG. 1, in which an end 12d of a long supply pipe 12c is placed directly on the bottom surface of the volatile solvent 12a, or an aeration pipe 12e made of a porous material that releases the enclosed gas as fine bubbles is attached to the end 12d.

The method of the agitator 14 agitating the volatile solvent 12a in the mixing tank 12 may be, for example, a method of rotating a predetermined agitator blade 14a at a predetermined rotation speed or a method of rotating an agitator using magnetic force, as in a magnetic stirrer, as shown in FIG. 1. Also, the ozone 12b may be agitated and finely divided by vibrating a predetermined vibrator placed in the volatile solvent 12a or irradiating the volatile solvent 12a with ultrasonic waves using a predetermined ultrasonic generator.

In addition, since the concentration of ozone 12b in the volatile solvent 12a is proportional to the blowing time of the ozone 12b, it is usually possible to obtain the target concentration of ozone 12b (target concentration) by adjusting the blowing time (sec).

The method for producing the ozone-containing composition according to the present invention includes a mixing step of mixing a volatile oily solvent, a volatile aqueous solvent, and ozone in a mixing tank 12 using the above-mentioned production apparatus 10, and a stirring step of stirring the mixture in the mixing tank 12 with a predetermined stirrer 14. It is possible to produce the ozone-containing composition of the present invention.

Here, in addition to the above, the method for producing an ozone-containing composition according to the present invention may be, for example, a method in which ozone is contained in a volatile oily solvent in advance, and the mixing step may involve putting a volatile oily solvent containing ozone and a volatile aqueous solvent into the mixing tank 12 as a mixture and mixing the volatile oily solvent, the volatile aqueous solvent, and the ozone. Also, for example, a method in which ozone is contained in a volatile aqueous solvent in advance, and the mixing step may involve putting a volatile aqueous solvent containing ozone and a volatile oily solvent into the mixing tank 12 as a mixture and mixing the volatile oily solvent, the volatile aqueous solvent, and the ozone. Furthermore, for example, the mixing step may involve putting a volatile oily solvent and ozone into the mixing tank 12 as a mixture and mixing the volatile oily solvent and ozone, and then adding and mixing the volatile aqueous solvent. The order of mixing the volatile oily solvent, the volatile aqueous solvent, and the ozone is not particularly limited.

### [EXAMPLES]

The following provides specific descriptions of Examples, Comparative Examples, and the like of the present invention; however, the scope of the present invention is not limited to these Examples and the like.

### (Production of ozone-containing composition)

The ozone-containing composition according to the present invention was produced using the producing apparatus shown in FIG. 1. As shown in the tables of Figures 4 to 8, the components were mixed to produce the ozone-containing compositions of Examples 1 to 41 and Comparative Examples 1 and 2. The values in the tables indicate the concentration (wt%). The concentration of ozone among the components was set within the range of 0.1 ppm to 5000.0 ppm by adjusting the blowing time. When the ozone concentration is "good", it means a low concentration of, for example, 0.1 ppm to 100.0 ppm, and when the ozone concentration is "oo", it means a high concentration of, for example, 100.0 ppm to 5000.0 ppm.

### (Evaluation method)

Next, the performance of the ozone-containing composition produced was evaluated. The performance evaluation items were (1) mosquito killing test, (2) acaricidal test, (3) anti-acaricidal test, (4) paclitaxel decontamination test, (5) 5-fluorouracil decontamination test, (6) volatility test, and (7) cockroach killing test.

### (1) Mosquito killing test

A glass cylinder with an inner diameter of 20 cm and a length of 43 cm was placed horizontally, one opening was blocked with a nylon mesh cloth, and the other opening was blocked with a nylon mesh cloth with sleeves. A certain number of mosquitoes (about 60 mosquitoes) were put into the cylinder, and an ozone-containing composition was directly sprayed from the nylon mesh cloth with sleeves toward the inside for 5 seconds. Five minutes and 60 minutes after spraying, the mosquitoes were transferred from the cylinder to a glass petri dish, and the number of dead mosquitoes was counted. The quotient obtained by dividing the number of dead mosquitoes by the number of put-in mosquitoes was calculated as the mosquito mortality rate (%) and ranked according to the following criteria. The evaluations of "o" and "oo" indicate that the product is acceptable. FIG. 2 shows a front view, a right side view, and a left side view immediately after spraying, showing an example of the results of the mosquito insecticidal test in Example 1.

### <Criteria>

oo: When the mosquito mortality rate is between 75% and 100%
o: When the mosquito mortality rate is between 50% and 75%
x: When the mosquito mortality rate is between 25% and 50%
xx: When the mosquito mortality rate is between 0% and 25%

### (2) Acaricidal Test

0.5 mL of ozone-containing composition was evenly dropped onto a filter paper 10 cm long and 5 cm wide, which was then folded in half to make a size of 5 cm x 5 cm. A specified number (several dozen) of live test pest mites were placed inside the folded part, and the three sides other than the fold were completely sealed with clips. After 24 hours, the clips were removed, the live mites were observed under a microscope, and the number of dead live mites was counted. The quotient obtained by dividing the number of dead mites by the number placed in was calculated as the mosquito mortality rate (%), and the product was ranked according to the following criteria. A rating of "o" or "oo" indicates that the product is acceptable.

### <Criteria>

great: When the mite mortality rate is between 75% and 100%
o: When the mite mortality rate is between 50% and 75%
x: When the mite mortality rate is between 25% and 50%
xx: When the mite mortality rate is between 0% and 25%

### (3) Anti-acaricidal test

A filter paper with a diameter of 40 mm on which a liquid of an ozone-containing composition was dropped was placed in a petri dish, and 50 mg (about 250 mites) of the test pest mite breeding medium was spread in the center with a diameter of about 3 cm. In addition, a mite breeding medium was placed in an empty petri dish in the center adjacent to the petri dish. The petri dish was immersed in saturated saline solution, kept at 25 degrees, and left for 24 hours. The movement of the mites was then observed. The value obtained by dividing the diameter of the mites (mite breeding medium) after the movement by the diameter of the mites before the movement (about 3 cm) was calculated as the mite repellency rate (%) and ranked according to the following criteria. The evaluations of "o" and "oo" are acceptable products. FIG. 3 is a photograph showing an example of the anti-mite test results in Reference Example 1 and Example 1. Reference Example 1 shows a control, which is a mixture of a volatile oily solvent and a volatile aqueous solvent that does not contain ozone. As shown in Fig. 3, in the anti-mite test, the diameter of the mites in Reference Example 1 is shown as a circle of about 3 cm, which is almost the same as the diameter of the mites before migration. On the other hand, the diameter of the mites in Example 1 is shown as a circle of 1 cm or less, which indicates that the diameter of the mites is reduced by the liquid of the ozone-containing composition that has a mite repellent effect.

### <Criteria>

oo: When the mite repellency rate is 0% to 25%
o: When the mite repellency rate is 25% to 50%
x: When the mite repellency rate is 50% to 75%
xx: When the mite repellency rate is 75% to over 100%

### (4) Paclitaxel decontamination test

10 µL of paclitaxel was dropped onto a stainless steel pad, which was then wiped once with a cleaning wipe soaked in 15 mL of water, and either sprayed with an ozone-containing composition liquid or wiped once with an ozone-containing composition wipe. 250 µL of extraction solution was then dropped onto the pad, and the solution was wiped off twice with filter paper. The wiped filter paper was then placed into a test tube, and the concentration (ng/mL) of residual paclitaxel was calculated using a liquid chromatograph mass spectrometer (LC-MS/MS), and ranked according to the following criteria. Evaluations of "o" and "oo" indicate that the product is acceptable.

### <Criteria>

oo: When the residual paclitaxel concentration is 0.00ng/mL to 0.30ng/mL
o: When the residual paclitaxel concentration is 0.30ng/mL to 0.60ng/mL
x: When the residual paclitaxel concentration is 0.60ng/mL to 0.90ng/mL
xx: When the residual paclitaxel concentration is 0.90ng/mL or higher

### (5) 5-fluorouracil decontamination test,

(4) Same as paclitaxel decontamination test. 10 µL of 5-fluorouracil (5-FU) was dropped onto a stainless steel pad, wiped once with a cleaning wipe soaked in 15 mL of water, and then sprayed with an ozone-containing composition liquid or wiped once with a wipe soaked in an ozone-containing composition. Then, 250 µL of extraction solution was dropped onto it, and the solution was wiped twice with filter paper. The wiped filter paper was then placed into a test tube, and the residual 5-FU concentration (ng/mL) was calculated using a liquid chromatograph mass spectrometer (LC-MS/MS), and ranked according to the following criteria. Evaluations of "o" and "oo" indicate that the product is acceptable.

### <Criteria>

oo: When the residual 5-FU concentration is 0.00ng/mL to 0.30ng/mL
o: When the residual 5-FU concentration is 0.30ng/mL to 0.60ng/mL
x: When the residual 5-FU concentration is 0.60ng/mL to 0.90ng/mL
xx: When the residual 5-FU concentration is 0.90ng/mL or higher

### (6) Volatility test

A specified amount of ozone-containing composition liquid (approximately 1 mL, the amount of one spray) was dropped onto a glass plate, then it was kept at 25 degrees and left to stand, and 10 minutes after dropping, the state of drying of the ozone-containing composition liquid was observed. The weight of the ozone-containing composition liquid after 10 minutes was divided by the amount dropped to calculate the drying rate (%), and the product was ranked according to the following criteria. Evaluations of "o" and "oo" are acceptable products.

### <Criteria>

oo: When the drying ratio is 0% to 25%
o: When the drying ratio is 25% to 50%
x: When the drying ratio is 50% to 80%
xx: When the drying ratio is 80% to 100%

### (7) Cockroach killing test

A cockroach container containing three cockroaches in a semi-transparent container with a diameter of about 13 cm, a height of about 12 cm, and a capacity of about 1.4 L was used as one treatment area. Next, an aerosol product was prepared by filling an aerosol container with an ozone-containing composition together with high-pressure gas (LPG, liquefied petroleum gas), and the aerosol product was sprayed for three seconds from a height of about 20 cm from the opening of the cockroach container. After that, the cockroach in the cockroach container was visually checked to see if it had stopped moving for about 30 seconds or if it had not moved even when the cockroach container was shaken to stimulate it. If the cockroach did not move, it was determined that it had died. At that time, the time of death from the time of spraying to the time the cockroach's death was confirmed was calculated, and it was ranked according to the following criteria. The evaluations of "o" and "oo" indicate that the product is acceptable.

### <Criteria>

oo: Death occurs within 5 minutes
o: Death occurs between 5 and 10 minutes
x: Death occurs between 10 and 30 minutes
xx: Death occurs within 30 minutes but does not occur

### (Evaluation Results)

As shown in FIG 4, Comparative Example 1 contains a volatile oil-based solvent and ozone, but does not contain a volatile aqueous solvent, so the ozone does not come into proper contact with the insect pest's trachea or the anticancer drug, and the evaluation results for (1) mosquito killing test, (2) acaricidal test, (3) anti-acaricidal test, (4) paclitaxel decontamination test, and (5) 5-fluorouracil decontamination test were all "x". Here, Comparative Example 1 had sufficient effects compared to the effects of general insecticides, but the product was not evaluated as being sufficiently effective according to the standards of the present invention, so it was rejected. In addition, in Comparative Example 2, although a volatile aqueous solvent and ozone were contained, a volatile oily solvent was not contained, and therefore the ozone itself was not dissolved in the liquid. As a result, the evaluation results of (1) mosquito killing test, (2) acaricidal property test, (3) anti-acaricidal property test, (4) paclitaxel decontamination test, and (5) 5-fluorouracil decontamination test were all marked as "X," and the product was unsuccessful.

On the other hand, in Examples 1 to 9, when the concentrations of the volatile oily solvent and the volatile aqueous solvent were within a specific concentration range, the evaluation results of (1) mosquito insecticidal test, (2) acaricidal test, (3) anti-mite test, (4) paclitaxel decontamination test, and (5) 5-fluorouracil decontamination test were "o" or "oo". (6) The evaluation result of the volatility test was "o" or "oo", and the product passed the test.

Next, as shown in FIG. 5, in Examples 10 to 18, even when the type of volatile oily solvent was any one of light isoparaffin, light liquid isoparaffin, hydrogenated isobutene, cyclic dimethyl silicone oil, or a mixture thereof, the evaluation results of (1) mosquito insecticidal test, (2) acaricidal test, (3) anti-acaricidal test, (4) paclitaxel decontamination test, and (5) 5-fluorouracil decontamination test were "oo", and the evaluation result of (6) volatility test was "o", and the product was acceptable.

Furthermore, as shown in FIG. 6, in Examples 19 to 29, even when the type of volatile aqueous solvent was any of volatile alcohols (isopropanol, ethanol, 1-pentanol), volatile ketones (acetone, methyl ethyl ketone, 2-hexanone), or mixtures thereof, the evaluation results of (1) mosquito insecticidal test, (2) acaricidal test, (3) anti-acaricidal test, (4) paclitaxel decontamination test, and (5) 5-fluorouracil decontamination test were "oo", and the evaluation result of (4) volatility test was "o", indicating that the product passed.

Furthermore, as shown in FIG. 7 , in Examples 30 to 36, even when a volatile oily solvent, a volatile aqueous solvent, and ozone were contained, and further, any of hydrogen peroxide, an alkaline agent such as sodium hydroxide, an acidic agent such as hydrochloric acid, a fragrance, and a colorant was added as an additive, the evaluation results of (1) mosquito killing test, (2) acaricidal test, (3) anti-mite test, (4) paclitaxel decontamination test, and (5) 5-fluorouracil decontamination test were "oo", and the evaluation result of (4) volatility test was "o", and the product was acceptable.

Furthermore, as shown in FIG. 8, in Examples 37 to 41, despite the low concentration of the volatile oily solvent and the low concentration of ozone, by adding hydrogen peroxide or sodium hydroxide as an additive, the evaluation results of (1) mosquito insecticidal test, (2) acaricidal test, (3) anti-mite test, (4) paclitaxel decontamination test, (5) 5-fluorouracil decontamination test, and (6) volatility test were "Excellent", indicating that the product passed. Hydrogen peroxide was particularly effective. This is thought to be because the oxidizing power of ozone was strengthened by adding an additive containing hydrogen peroxide.

Here, when the evaluation results of the (7) cockroach insecticidal test were examined, the aerosol product containing only LPG in Reference Example 2 did not kill cockroaches, resulting in an evaluation result of "xx". Furthermore, the aerosol product containing the ozone-free composition (volatile oily solvent and volatile aqueous solvent) and LPG in Reference Example 3 took more than 10 minutes to kill cockroaches, resulting in an evaluation result of "x". FIG. 8 shows a photograph showing an example of the cockroach insecticidal test in Example 1. On the other hand, the aerosol product containing the ozone-containing composition in Example 1 killed cockroaches immediately, resulting in an evaluation result of "oo", indicating that the product passed the evaluation.

### [INDUSTRIAL APPLICABILITY]

As described above, the ozone-containing composition and the method for producing the ozone-containing composition according to the present invention are useful not only in the fields of insecticide and pest repellent and the decontamination and decomposition of anticancer drugs, but also in the fields of hygiene, medicine, agriculture, dairy farming, etc., and are effective as the ozone-containing composition and the method for producing the ozone-containing composition that can obtain an insecticidal effect, an insect pest repellent effect, and an effect of decontaminating and decomposing an anticancer drug, can be safe for the human body, and can be easily post-treated after application.

### [REFERENCE SIGNS LIST]

10 Producing Apparatus
11 Generator Unit
12 Mixing Tank
12a Volatile Solvent
12b Ozone
13 Supply Unit
14 Agitator

## Claims

1. An ozone-containing composition comprising:
a volatile oily solvent being present in a concentration ranging from 1 wt% to 99 wt% based on the total composition;
a volatile aqueous solvent being present in a concentration ranging from 1 wt% to 99 wt% based on the total composition; and
ozone;
wherein:
the volatile oily solvent is a volatile liquid paraffin having a boiling point of 170 degrees and being lipophilic, a volatile silicone oil having a boiling point ranging from 170 degrees to 200 degrees and being lipophilic, or a mixture of the volatile liquid paraffin and the volatile silicone oil;
the volatile liquid paraffin is one or more volatile liquid paraffins selected from the group consisting of light isoparaffin, light liquid isoparaffin, and hydrogenated polyisobutene;
the volatile silicone oil is a volatile silicone oil containing cyclic dimethyl silicone oil;
the volatile aqueous solvent is a volatile alcohol having a boiling point of 150 degrees or less and being hydrophilic and lipophilic, a volatile ketone having a boiling point of 150 degrees or less and being hydrophilic and lipophilic, or a mixture of the volatile alcohol and the volatile ketone;
the volatile alcohol comprises ethanol, isopropyl alcohol, or 1-pentanol;
the volatile ketone comprises methyl ethyl ketone, or 2-hexanone; and
the ozone-containing composition is for use in killing insects, repelling pests, or decontaminating and decomposing anticancer drugs.

2. The ozone-containing composition according to claim 1, wherein:
the volatile oily solvent is present in a concentration ranging from 10 wt% to 90 wt% based on the total composition; and
the volatile aqueous solvent being present in a concentration ranging from 10 wt% to 90 wt% based on the total composition.

3. The ozone-containing composition according to claim 1, wherein:
the ozone-containing composition further comprises an additive selected from an oxidizing agent including a hydrogen peroxide, a reducing agent, an alkaline agent, and an acidic agent.

4. The ozone-containing composition according to claim 1, wherein:
the ozone-containing composition further comprises an additive including a hydrogen peroxide.

5. The ozone-containing composition according to claim 1, wherein:
the ozone-containing composition further comprises an additive including a hydrogen peroxide or an alkaline agent of sodium hydroxide; and
the concentration of the additive is set within a range of 0.1 wt% to 10.0 wt% based on the total composition.

6. The ozone-containing composition according to claim 1, wherein:
the mixing weight ratio (-) of the volatile oily solvent to the volatile aqueous solvent is within the range of 0.01 to 99.00.

7. The ozone-containing composition according to claim 1, wherein:
the volatile oily solvent is present in a concentration ranging from 10 wt% to 90 wt% based on the total composition;
the volatile aqueous solvent being present in a concentration ranging from 10 wt% to 90 wt% based on the total composition; and
the mixing weight ratio (-) of the volatile oily solvent to the volatile aqueous solvent is within the range of 0.01 to 99.00.

8. The ozone-containing composition according to claim 1, wherein:
the volatile oily solvent is present in a concentration ranging from 10 wt% to 90 wt% based on the total composition;
the volatile aqueous solvent being present in a concentration ranging from 10 wt% to 90 wt% based on the total composition;
the mixing weight ratio (-) of the volatile oily solvent to the volatile aqueous solvent is within the range of 0.01 to 99.00; and
the ozone-containing composition further comprises an additive including a hydrogen peroxide or an alkaline agent of sodium hydroxide.

9. The ozone-containing composition according to claim 1, wherein:
the volatile oily solvent is present in a concentration ranging from 10 wt% to 90 wt% based on the total composition;
the volatile aqueous solvent being present in a concentration ranging from 10 wt% to 90 wt% based on the total composition;
the mixing weight ratio (-) of the volatile oily solvent to the volatile aqueous solvent is within the range of 0.01 to 99.00;
the ozone-containing composition further comprises an additive including a hydrogen peroxide or an alkaline agent of sodium hydroxide; and
the concentration of the additive is set within a range of 0.1 wt% to 10.0 wt% based on the total composition.

10. A method for producing an ozone-containing composition comprising:
a mixing step of introducing a volatile oily solvent with a concentration in the range of 1 wt% to 99 wt% based on the total composition, a volatile aqueous solvent with a concentration in the range of 1 wt% to 99 wt% based on the total composition, and ozone into a predetermined mixing tank as a mixture of the ozone-containing composition and mixing them; and
a stirring step of stirring the mixture in the mixing tank using a predetermined stirrer;
wherein:
the volatile oily solvent is a volatile liquid paraffin having a boiling point of 170 degrees and being lipophilic, a volatile silicone oil having a boiling point ranging from 170 degrees to 200 degrees and being lipophilic, or a mixture of the volatile liquid paraffin and the volatile silicone oil;
the volatile liquid paraffin is one or more volatile liquid paraffins selected from the group consisting of light isoparaffin, light liquid isoparaffin, and hydrogenated polyisobutene;
the volatile silicone oil is a volatile silicone oil containing cyclic dimethyl silicone oil;
the volatile aqueous solvent is a volatile alcohol having a boiling point of 150 degrees or less and being hydrophilic and lipophilic, a volatile ketone having a boiling point of 150 degrees or less and being hydrophilic and lipophilic, or a mixture of the volatile alcohol and the volatile ketone;
the volatile alcohol comprises ethanol, isopropyl alcohol, or 1-pentanol;
the volatile ketone comprises methyl ethyl ketone, or 2-hexanone; and
the ozone-containing composition is for use in killing insects, repelling pests, or decontaminating and decomposing anticancer drugs.
